# EUROPEAN PATENT SPECIFICATION

(11) **EP 0 438 531 B1**
(45) Date of publication and mention of the grant of the patent: **12.01.1994**
(21) Application number: 89912812.8
(22) Date of filing: 09.10.1989
(51) Int. Cl.: A61K 37/30, A61K 9/06

(54) **PHARMACEUTICAL COMPOSITIONS COMPRISING CALCITONIN FOR INTRANASAL ADMINISTRATION AND A SPRAY UNIT FOR THE ADMINISTRATION OF THE SAME**
PHARMAZEUTISCHE ZUSAMMENSETZUNGEN MIT GEHALT AN CALCITONIN ZUR INTRANASALEN VERABREICHUNG UND EINE SPRAY-EINHEIT ZUR VERABREICHUNG DERSELBEN
COMPOSITIONS PHARMACEUTIQUES COMPRENANT DE LA CALCITONINE POUR ADMINISTRATION INTRANASALE ET UNITE DE VAPORISATION SERVANT A L'ADMINISTRATION D'UNE TELLE COMPOSITION

(30) Priority: 11.10.1988 IT 2225788; 08.11.1988 IT 2254888
(43) Date of publication of application: 31.07.1991
(73) Proprietor: SCHIAPPARELLI SALUTE S.p.A., I-20126 Milano (IT)
(72) Inventor: MARDENTE, Salvatore, I-20124 Milano (IT); CORNELI, Rodolfo, I-20124 Milano (IT); CARAZZONE, Marilena, I-20124 Milano (IT)
(74) Representative: Perani, Aurelio
(86) International application number: EP8901186
(87) International publication number: WO9003796

(56) References cited:
- EP-A- 0 156 772
- EP-A- 0 193 372
- GB-A- 2 114 001

## Description

The present invention relates to pharmaceutical compositions comprising calcitonin and to a spray unit for the intranasal administration of said compositions.

More particularly, the invention relates to pharmaceutical compositions whose active ingredient is calcitonin,which compositions are free from preservants and surfactants.

Still more particularly, the invention relates to pharmaceutical compositions for intranasal administration, comprising calcitonin - preferably salmon calcitonin - dissolved in physiological saline adjusted to pH 3.5-4.5 by means of hydrochloric acid.

Calcitonins are known to be of basic importance in the treatment of osteoporosis, Paget's disease, hypercalcemia and similar pathological conditions. Calcitonins are also known to undergo degradation easily, due to the polypeptide nature thereof: as a consequence, they cannot be administered by the oral route. On the other hand, parenteral administration of calcitonins by injection involves remarkable disadvantages, particularly painful reactions in the patient.

Intranasal administration of polypeptide drugs is also widely described in literature; see, e.g.,Felber et al., Experientia, 1969, page 1195; Gennser et al., Lancet, 1974, page 865; Greenberg et al., Antimicrobial Agents and Chemotherapy, 1978, page 596; Bergquist et al., Lancet, 1979, page 215; Pontiroli et al., British Medical Journal, 1982, page 303, besides a number of patents or patent applications concerning intranasal administrations of insulin (EP 94157), vasopressin (Japanese Patent applications 55066-517, 55055-120), polypeptides of various nature (German Patents 2.256.445 and 2.758.483, EP 252, Belgian Patent 860.717, South african Patent 68/4241).

Intranasal administration of calcitonin is in its turn disclosed in various patents. Thus, italian Patent 1.172.324 claims galenic compositions comprising calcitonin characterized in that they contain, besides the active ingredient, benzalkonium chloride or a surfactant, particularly a non-ionic surfactant, or both the above additives. Higher alkanols or sterol polyoxyalkylene ethers are particularly claimed as surfactants.

Both surfactants and benzalkonium chloride are indicated to be necessary ingredients to provide a good calcitonin bioavailability, that is to assure the effective adsorption thereof by nasal mucosa, while benzalkonium chloride would also act as a preservant against pathogen or undesirable micro-organisms.

Similar compositions still containing a quaternary ammonium salt (benzalkonium chloride, cetyltrimethylammonium bromide) are claimed in EP-A-193.372.
Japanese Patent Application 61126-034 discloses on its turn nasal formulations of calcitonin containing absorption-increasing agents such as glucose and/or glucosamine;the need for an absorption adjuvant is also stressed by Japanese Patent Application 61118-325, which proposes to this purpose the addition of amino acids, and by EP-A-183.527, which on the contrary suggests the use of benzyl alcohol, ethanol, salicylic acid, capronic acid or polyethylene glycol as absorption enhancers. Moreover, EP-A-111 841 and 115 627 claim intranasal compositions containing calcitonin in admixture with surfactants, among which biliary acids and benzalkonium chloride are disclosed.

Nevertheless, the presence of the latter - and generally of quaternary ammonium salts - is not satisfactory due to the possible undesired effects thereof (Am J Ophthalmol-1988, 105 (6) p 670-3; Contact Dermatitis-1987, 17 (1) p 41-2; Cutis-1987, 39 (5) p 381-3). Therefore, it would be better to provide intranasal compositions of calcitonin free from said salts; however, according to some of the above mentioned documents, said compositions would suffer from poor bioavailability.

All the patent documents reported above completely agree on the need to enhance said bioavailability by means of surfactants of various nature which promote calcitonin absorption through nasal mucosa.

Therefore, it is really surprising the finding by the Applicant that intrasal compositions of calcitonin containing surfactants of various type, or anyhow agents intended to increase absorption by the nasal mucosa, and compositions containing calcitonin alone in a substantially physiological aqueous solution of sodium chloride, practically show no differences in bioavailability nor in preservability.

Therefore, an object of the present invention is provided by pharmaceutical formulations for intranasal administration comprising:
a) a therapeutically effective amount of calcitonin, dissolved in
b) an about 0.9% NaCl solution in purified water adjusted to pH 3.5-4.5.

Preferably, according to the invention, the compositions consist of:
a) a therapeutically effective amount of salmon calcitonin dissolved in
b) an about 0.9% NaCl solution in purified water, adjusted to pH 3.5-4.5 by means of hydrochloric acid.

Moreover, the compositions according to the invention preferably contain 100 to 5.000 I.U., advantageously 250 to 2.000 I.U., of calcitonin per ml of composition.

The advantage from the compositions of the invention, compared to the ones of the prior art, is evident not only since it is possible to avoid quaternary ammonium salts, but also because the addition of the up-to-now suggested calcitonin-absorption enhancer agents can also be avoided.

Said agents, in fact, involve an increase in costs of starting materials and working.

According to a preferred embodiment, the present invention provides also a spray unit intended for spray administration of the above formulations, comprising a bottle (1) for enclosing the composition and a dosing pump fitted to the mouth of the bottle, this spray unit being further characterized in that a head space in said bottle is filled with nitrogen under pressure.

Spray bottles of the above type are known but they suffer from a number of disadvantages in that, for example, problems arise in ensuring tightness to a liquid enclosed therein and especially to any added fat.

The spray bottle according to this invention permits avoiding the problems referred to above and this, especially, owing to a particular construction of its pump which has no suction/delivery valve means (usually of the ball type) associated thereto, and which is designed so as to prevent, in both the inoperative and operative conditions, the content of the bottle from being ejected through the pump.

The pump is, moreover, integrally equipped with a collar packing tightly fitting to the mouth of the bottle thereby to provide a hermetical seal preventing any exchange of gas with the outside, in both the inward and outward direction.

A head space in the bottle is charged with nitrogen which provides an overpressure of 0.2-0.3 bar. This over-pressure is to compensate for liquid withdrawal during spray administration thereby ensuring that the whole of the bottle content is throughly utilized without back-flow of air occurring from the outside. This is of particular benefit if it is thought that the contact of liquid pharmaceutical composition in the bottle with air may result in degradation of the active principle of the composition as well as in bacterial contamination of this latter and, thus, in damage to a person making use thereof.

The bottle is provided with a sump bottom and has a capacity of about 7 ml, and the precompression dosing pump delivers about 100 microliters at each operation which corresponds to a therapeutically effective dose of active principle.

The pump comprises a hollow casing defining a plurality of chambers of varying cross-sections, which is located in the bottle neck with the interposition of said collar packing, and which is secured to the bottle by the aid of a capsule having a bottom member that is rolled around an annular external rim of the bottle neck.

The hollow pump casing has a hollow piston received therein which is spring urged towards a valve seat formed in a piston rod that extends upwardly out of the top of bottle.

Depressing the piston rod causes discharge of liquid enclosed in a toric chamber which is defined between the external surface of the piston and the internal surface of the largest section of pump casing.

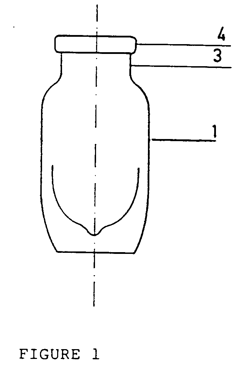

Further features of the spray unit of the invention will be better understood when reading the following detailed description of one embodiment thereof shown in the accompanying drawings, wherein
Figure 1 is a side elevational view of a bottle embodying the invention; and
Figure 2 is a cross sectional view shoving on a larger scale a pump as fitted to the neck of the bottle in figure 1.

Referring to the above figures, generally designated with 1 is a glass bottle having a sump bottom 2 and a capacity of preferably about 7 ml.

The bottle 1 is, in a known manner, provided with a neck 3 and the neck 3 has an external raised anular rim, 4 at its top.

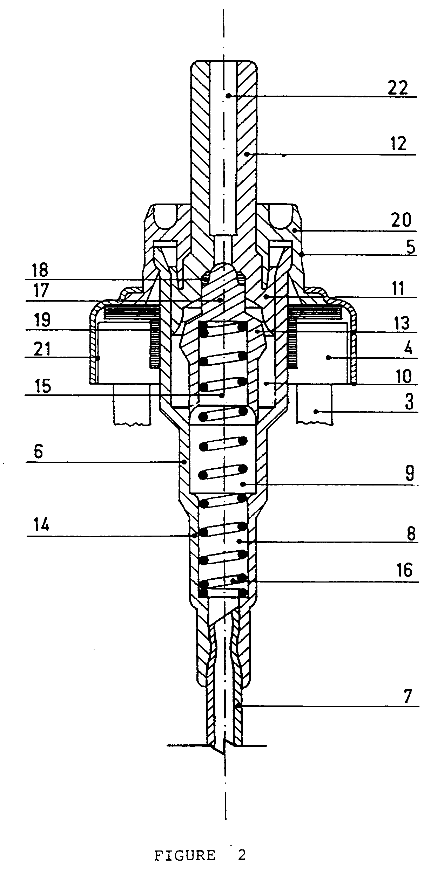

Mounted and sleeved to the bottle 1 is a precompression dosing pump 5 (figure 2) which is designed to deliver about 100 microliters per operation.

The pump 5 comprises a hollow casing 6 fitted in the bottle 1 and carrying at a lower part thereof a plunger member 7.

The hollow casing 6 defines, as seen from bottom to top, three chambers 8, 9 and 10 of increasing sections, and the uppermost of these chambers is arranged to sealingly receive the base 11 of a hollow pistons rod 12 that extends upwardly to project out the top of bottle 1.

Arranged in the pump casing 6 is a piston 13 which is sealingly slidable in the inside of chamber 9 and which is normally urged upwardly by a spring 14 partially located at one end in a recess 15 in piston 13 and resting, at its other end, against an annular shoulder 16 provided in the bottom of the lowermost chamber 8 of casing 6.

The head 17 of piston 13 functions as a valve means and is normally urged by spring 14 towards a valve seat 18 formed in base 11 of piston rod 12.

A collar packing 19 of silicone rubber is interposed between the pump casing 6 and the mouth of bottle 1 and serves as a hermetical seal which besides preventing liquid from flowing out of the bottle, also keeps any exchange of gas from occurring with the outside in neither directions.

The bottle 1 has a capsule 20 of plastic material fitted over its mouth, this capsule being centrally provided with a hole for passage of piston rod 12 and being secured to the bottle by having an aluminium bottom 21 rolled around the annular raised rim 4 of the bottle.

A head space within the bottle 1 is charged with nitrogen to an overpressure of 0.2-0.3 bar.

This overpressure is for the purpose of compressing withdrawal of liquid during utilization so as to ensure thorough use of the whole bottle content, without air backflow from the outside, and, thus, also to ensure that a nitrogen atmosphere is maintained during operation without time limitations.

The nitrogen atmosphere in the bottle is obtained by flushing with nitrogen the empty bottle, distributing the solution, discharging gas from the bottle by vacuum and replacing it by nitrogen, mounting and sleeving the pump, producing overpressure through the pump. All of these operations are accomplished under nitrogen atmosphere.

In this manner, air is nearly thoroughly removed form a head space in the bottle, which head space practically only consists of nitrogen that is maintained over the whole period of validity of product.

In order to use the spray bottle according to the invention, the hollow piston rod 12 is depressed thereby causing the piston 13 to move down against the force of spring 14.

The downward movement of piston 13 results in the toric chamber 10 reducing its volume so that liquid enclosed therein will tend to flow into the head 17 of piston 13 and the valve seat 18 to be then issued as a spray from a passage-way 22 in hollow piston rod 12.

Releasing the piston rod 12 causes the piston 13 and, thus, the piston rod 12 to be urged by spring 14 upwardly to a position as shown in figure 2 which results in further liquid flowing into the toric chamber 10.

The quantity of liquid that is pumped at each delivery, which quantity nearly corresponds to the volume of toric chamber 10, is about 100 microliters.

From the above, it should be apparent that the particular construction of the described pump, which has no suction/delivery valve (usually of the ball type), prevents the bottle content from being ejected through the pump in both the inoperative and operative conditions.

The compositions of the invention were compared with compositions prepared according to the prior art; particularly the pharmaco-kinetic following intranasal administration of the same dosages (100 I.U.) of salmon calcitonin (salcatonin) in the formulations A and B, in 12 subjects each time:

| | | |
|---|---|---|
| A) | Salcatonin | I.U. 100 |
| | Sodium chloride | mg 0.9 |
| | 1N hydrochloric acid , q. s. to | pH 4.0 |
| | Purified water, q. s. to | ml 0.1 |

| | | |
|---|---|---|
| B) | Salcatonin | U.I. 100 |
| | benzalkonium chloride | mg 0.0091 |
| | 1N hydrochloric | mg 0.77 |
| | Purified water, q. s.. to | pH 4.0 |
| | Treatment | ml 0.1 |

Salcatonin plasma concentrations (12 subject mean ± standard error) at various times from administration, are reported in the following Table:

| Minutes | A (pc/ml) | B (pc/ml) |
|---|---|---|
| 0 | 0 | 0 |
| 5 | 25 | 25 |
| 10 | 52 | 55 |
| 20 | 68 | 72 |
| 30 | 38 | 45 |
| 40 | 22 | 20 |
| 50 | 15 | 12 |
| 60 | 10 | 5 |
| 120 | 0 | 0 |

The AUC (area under curve) is 1663 in case of A and 2108 in case of B. T/2β is 12.2 minutes and 11.7 minutes, respectively.

The difference between the two curve types is not significant.

Completely analogous results are obtained by comparing the compositions of the invention with compositions containing, instead of benzalkonium chloride, a cholesterol polyoxyethylene ether or respectively ethanol, salicylic acid and sodium taurocholate.

The following examples illustrate the invention in more detail.

### EXAMPLE 1

Salcatonin (500.000 I.U.) is dissolved in 850 ml of depurated water containing 9 g of analytically pure sodium chloride;pH is adjusted to 4.0 by means of analytically pure 1N HCl and the solution is diluted to 1.000 ml by adding purified water. The solution is filtered on a 0.2 µm filter and distributed in 5 ml containers as disclosed in the annexed figures, provided with a doser delivering 0.1 ml doses each time, equal to 50 I.U. of salcatonin.

### EXAMPLE 2

The procedure of example 1 is followed, but using a double amount of salcatonin (1.000.000 I.U.). The solution is distributed in suitable 2.5 ml containers as disclosed in the annexed figures delivering 0.1 ml doses each time, equal to 100 U.I. of salcatonin.

## Claims

1. Liquid pharmaceutical compositions for intranasal administration, consisting of a therapeutically effective amount of calcitonin dissolved in a sodium chloride solution in depurated water adjusted to pH 3.5-4.5.

2. Liquid pharmaceutical compositions as claim 1, in which calcitonin is present in concentrations of 100 to 5.000 I.U. per ml of composition.

3. Pharmaceutical compositions as claimed in claim 2, in wich calcitonin is present in concentrations of 250 to 2.000 I.U. per ml of composition.

4. Pharmaceutical compositions as claimed in claim 1 to 3, in which pH is adjusted to 3.5-4.5 by addition of hydrochloric acid.

5. Pharmaceutical compositions as claimed in claim 1 to 4, containing salmon calcitonin (salcatonin) as the calcitonin.

6. A liquid pharmaceutical composition for intranasal administration, having the following composition:
| | |
|---|---|
| salcatonin | U.I. 500 |
| sodium chloride | mg 9 |
| hydrochloric acid q. s. to | pH 4.0 |
| purified water q. s. to | ml 1.0 |

7. A liquid pharmaceutical composition for intranasal administration, having following composition.
| | |
|---|---|
| salcatonin | U.I.1000 |
| sodium chloride | mg 9 |
| hydrochloric acid q. s. to | pH 4.0 |
| purified water q.s. to | ml 1.0 |

8. A spray unit for spray administration of calcitonin by intranasal route, comprising a bottle (1) containing a pharmaceutical liquid composition consisting of a therapeutically effective amount of calcitonin dissolved in a sodium chloride solution in purified water, adjusted to pH 3.5-4.5, and a pump (5) mounted and sleeved to said bottle, the pump (5) comprising a hollow casing (6) formed of a plurality of sections, a piston (13) arranged to operate against the opposition of a spring (14), and a hollow piston rod (12) projecting out the top of bottle (1), characterized in that between the piston (13) and the hollow casing (6) there is defined a toric chamber (10) whose reduction in volume, as a result of the piston rod (12) being acted upon, causes the liquid in said chamber (10) to pass between the piston head (17) and a valve seat (18) formed in a base (11) of piston rod (12), the liquid then being permitted to flow out through a passageway (22) in hollow piston rod (12), and in that a head space in bottle (1) is filled with nitrogen under pressure.

9. The spray unit according to claim 8, wherein said head space in bottle (1) is filled with nitrogen to an overpressure of 0.2-0.3 bar.

10. The spray unit according to claim 8 or 9, wherein a collar packing (19) is arranged between the hollow casing (6) of pump (5) and the mouth of bottle (1) and functions as a hermetic seal for preventing gas from flowing into, or out the bottle.

11. The spray unit according to any one of claims 8 to 10, wherein the bottle (1) has a capacity of about 7 ml and the dosing pump (5) delivers about 100 microliters at each operation, which corresponds to nearly the volume of the toric chamber (10).

12. A pharmaceutical packaging consisting of the compositions of claims 1-7 dosed in the spray units of claims 8-11.

## Patentansprüche

1. Flüssige pharmazeutische Zusammensetzungen zur intranasalen Verabreichung, bestehend aus einer therapeutisch wirksamen Menge von Calcitonin, gelöst in einer Natriumchloridlösung in gereinigtem Wasser, eingestellt auf einen pH-wert zwischen 3,5 - 4,5.

2. Flüssige pharmazeutische Zusammensetzungen nach Anspruch 1, dadurch gekennzeichnet, daß Calcitonin in Konzentrationen von 100 bis 5000 i. U. pro ml der Zusammensetzung vorliegt.

3. Pharmazeutische Zusammensetzungen nach Anspruch 2, dadurch gekennzeichnet, daß Calcitonin in Konzentrationen von 250 bis 2000 i. U. pro ml der Zusammensetzung vorliegt.

4. Pharmazeutische Zusammensetzungen nach den Ansprüchen 1 bis 3, dadurch gekennzeichnet, daß der pH-Wert durch Zugabe von Salzsäure auf 3,5 - 4,5 eingestellt wird.

5. Pharmazeutische Zusammensetzungen nach den Ansprüchen 1 bis 4, dadurch gekennzeichnet, daß diese Lachs-Calcitonin (Salcatonin) als Calcitonin enthalten.

6. Eine flüssige pharmazeutische Zusammensetzung zur intranasalen Verabreichung, dadurch gekennzeichnet, daß sie die folgende Zusammensetzung hat:
| | |
|---|---|
| Salcatonin | i.U. 500 |
| Natriumchlorid | mg 9 |
| Salzsäure q. s. auf | pH 4,0 |
| gereinigtes Wasser q. s. auf | ml 1,0 |

7. Eine flüssige pharmazeutische Zusammensetzung zur intranasalen Verabreichung, dadurch gekennzeichnet, daß sie die folgende Zusammensetzung hat:
| | |
|---|---|
| Salcatonin | i.U. 1000 |
| Natriumchlorid | mg 9 |
| Salzsäure q. s. auf | pH 4,0 |
| gereinigtes Wasser q. s. auf | ml 1,0 |

8. Sprüheinheit zur Verabreichung von Calcitonin auf intranasalem Wege als Spray, umfassend eine Flasche (1), welche die pharmazeutische flüssige Zusammensetzung, bestehend aus einer therapeutisch wirksamen Menge Calcitonin gelöst in einer Natriumchlorid-Lösung in gereinigtem Wasser, eingestellt auf einen pH-Wert zwischen 3,5 - 4,5, und eine Pumpe (5), welche auf diese Flasche gesteckt und mit dieser fest verbunden ist, wobei die Pumpe (5) ein aus einer Vielzahl von Abschnitten gebildetes hohles Gehäuse (6), einen gegen die Feder (14) arbeitenden Kolben (13) und eine hohle, aus dem Oberteil der Flasche (1) führende Kolbenstange (12) umfasst, dadurch gekennzeichnet, daß zwischen dem Kolben (13) und dem hohlen Gehäuse (6) eine definierte torusförmige Kammer (10), deren durch die Wirkung der Kolbenstange (12) erzielte Volumenverminderung die Flüssigkeit in genannter Kammer (10) veranlaßt, zwischen dem Kolbenkopf (17) und einem Ventilsitz (18), welcher an der Basis (11) der Kolbenstange (12) gebildet wird, hindurchzutreten, und der Flüssigkeit gestattet, durch einen Durchlass (22) in der hohlen Kolbenstange (12) auszufließen und daß ein Flüssigkeitsüberstand in der Flasche (1) unter Druck mit Stickstoff gefüllt ist.

9. Sprayeinheit nach Anspruch 8, dadurch gekennzeichnet, daß der Flüssigkeitsüberstand in der Flasche (1) mit Stickstoff bei einem Überdruck von 0,2 - 0,3 bar gefüllt ist.

10. Sprayeinheit nach Anspruch 8 oder 9, dadurch gekennzeichnet, daß eine Kragenpackung (19) zwischen dem hohlen Gehäuse (6) der Pumpe (5) und der Öffnung der Flasche (1) angeordnet ist, welche als hermetische Dichtung dafür sorgt, daß kein Gas in die Flasche hinein oder aus der Flasche heraus strömen kann.

11. Sprayeinheit nach irgendeinem der Ansprüche 8 bis 10, dadurch gekennzeichnet, daß die Flasche (1) ein Volumen von etwa 7 ml aufweist und die Dosierpumpe (5) etwa 100 Microliter bei jeder Betätigung abgibt, welches in etwa dem Volumen der torusförmigen Kammer (10) entspricht.

12. Eine pharmazeutische Packung, bestehend aus einer Zusammensetzung der Ansprüche 1 -7, welche in einer Sprayeinheit der Ansprüche 8 - 11 dosiert ist.

## Revendications

1. Compositions liquides pharmaceutiques pour une administration intranasale, consistant en une quantité efficace sur le plan thérapeutique de calcitonine dissoute dans une solution de chlorure de sodium dans de l'eau purifiée ayant un pH ajusté à 3,5-4,5.

2. Compositions liquides pharmaceutiques selon la revendication 1, dans lesquelles la calcitonine est présente à des concentrations de 100 à 5.000 U.I. par ml de composition.

3. Compositions pharmaceutiques selon la revendication 2, dans lesquelles la calcitonine est présente à des concentrations de 250 à 2.000 U.I. par ml de composition.

4. Compositions pharmaceutiques selon les revendications 1 à 3, dans lesquelles le pH est ajusté à 3,5-4,5 par addition d'acide chlorhydrique.

5. Compositions pharmaceutiques selon les revendications 1 à 4, contenant de la calcitonine de saumon (salcatonine) en tant que calcitonine.

6. Composition pharmaceutique liquide pour une administration intranasale, ayant la composition suivante :
| | |
|---|---|
| salcatonine | 500 U.I. |
| chlorure de sodium | 9 mg |
| acide chlohydrique q.s.p. | pH 4,0 |
| eau purifiée q.s.p. | 1,0 ml |

7. Composition pharmaceutique liquide pour une administration intranasale, ayant la composition suivante :
| | |
|---|---|
| salcatonine | 1000 U.I. |
| chlorure de sodium | 9 mg |
| acide chlorhydrique q.s.p. | pH 4,0 |
| eau purifiée q.s.p. | 1,0 ml |

8. Unité de pulvérisation pour une administration par pulvérisation de calcitonine par voie intranasale, comprenant un flacon (1) contenant une composition liquide pharmaceutique consistant en une quantité efficace sur le plan thérapeutique de calcitonine dissoute dans une solution de chlorure de sodium dans de l'eau purifiée, dont le pH est ajusté à 3,5-4,5, et une pompe (5) montée et gaînée sur ledit flacon, la pompe (5) comprenant un boîtier creux (6) formé de plusieurs sections, un piston (13) installé pour fonctionner à l'encontre de l'action d'un ressort (14), et une tige de piston creuse (12) dépassant de la partie supérieure du flacon (1), caractérisée en ce que, entre le piston (13) et le boîtier creux (6), il existe une chambre torique (10) dont la réduction de volume, sous l'effet d'une action exercée sur la tige de piston (12), provoque le passage du liquide dans ladite chambre (10) entre la tête de piston (17) et un siège de valve (18) formé dans une embase (11) de la tige de piston (12), le liquide étant ainsi amené à sortir par un orifice de passage (22) ménagé dans la tige de piston creuse (12), et en ce qu'un espace libre dans le flacon (1) est rempli d'azote sous pression.

9. Unité de pulvérisation selon la revendication 8, dans laquelle ledit espace libre dans le flacon (1) est rempli d'azote à une surpression de 0,2-0,3 bar.

10. Unité de pulvérisation selon la revendication 8 ou 9, dans laquelle une garniture d'étanchéité à collet (19) est installée entre le boîtier creux (6) de la pompe (5) et le goulot du flacon (1) et fonctionne comme un dispositif d'étanchéité hermétique pour empêcher du gaz de s'écouler à l'intérieur du flacon ou hors de celui-ci.

11. Unité de pulvérisation selon l'une quelconque des revendications 8 à 10, dans laquelle le flacon (1) a une capacité d'environ 7 ml et la pompe doseuse (5) délivre environ 100 microlitres à chaque actionnement, ce qui correspond approximativement au volume de la chambre torique (10).

12. Conditionnement pharmaceutique consistant en les compositions des revendications 1 à 7 dosées dans les unités de pulvérisation des revendications 8 à 11.
